# EUROPÄISCHE PATENTSCHRIFT

(11) **EP 1 089 970 B1**
(45) Veröffentlichungstag und Bekanntmachung des Hinweises auf die Patenterteilung: **07.04.2004**
(21) Anmeldenummer: 99938207.0
(22) Anmeldetag: 22.06.1999
(51) Int. Cl.: C07C 405/00, A61K 31/557

(54) **AKTIVKOHLEFILTER ALS OXIDATIONSINHIBITOR BEI PROSTAN-DERIVATEN**
OXIDATION INHIBITORS FOR PROSTANE DERIVATIVES
INHIBITEUR D'OXYDATION UTILISE POUR LE TRAITEMENT DE DERIVES DE PROSTANE

(30) Priorität: 22.06.1998 DE 19828881
(43) Veröffentlichungstag der Anmeldung: 11.04.2001
(73) Patentinhaber: SCHERING AKTIENGESELLSCHAFT, 13342 Berlin (DE)
(72) Erfinder: WAGNER, Torsten, D-13127 Berlin (DE); WESSEL, Martin, D-14199 Berlin (DE); LIPP, Ralph, D-14169 Berlin (DE); IFFERT, Bernd, D-13465 Berlin (DE); MICHEL, Heinrich, D-13589 Berlin (DE); WESTERMANN, Jürgen, D-12161 Berlin (DE); DAHL, Helmut, D-13465 Berlin (DE); SKUBALLA, Werner, D-13465 Berlin (DE)
(86) Internationale Anmeldenummer: PCT/EP1999/004278
(87) Internationale Veröffentlichungsnummer: WO 1999/067212

(56) Entgegenhaltungen:
- EP-A- 0 055 208
- EP-A- 0 369 463
- WO-A-87/05294
- WO-A-88/07037
- WO-A-99/43303
- DE-A- 3 816 801
- STEINER T. ET AL: "A side chain of diastereomeric iloprost protudes from the cage in the complex with cyclomaltoheptaose (b-cyclodextrin): cristal structure of (b-cyclodextrin)2 iloprost. 20.5 H2O" CARBOHYDRATE RESEARCH, Bd. 192, 1989, Seiten 43-49, XP000867417

## Beschreibung

Die Erfindung betrifft die Verwendung von einem Aktivkohlefilter als Oxidationsinhibitor für die Umgebungsluft bei der Verarbeitung von Prostan - Derivaten, dabei bevorzugt während der feuchten Phase.

### Stand der Technik

Der Wirkstoff "Iloprost-β-Cyclodextrin" trägt die systematische Bezeichnung 5-(*E*)-(1*S*,5*S*,6*R*,7*R*)-7-hydroxy-6-[(*E*)-(3*S*,4R*S*)-3-hydroxy-4-methyl-1-octen-6-inyl]bicyclo[3.3.0]octan-3-yliden-pentansäure als β-Cyclodextrin-Einschlußverbindung. Der Wirkstoff wird durch die folgenden Schritte zu Rohpellets hergestellt.
(i) Mischen von dem Wirkstoff Iloprost-β-Cyclodextrin und den Hilfsstoffen Lactose und Avicel,
(ii) Pelletierung mit etwa 20,5% Wasser,
   - Schnellmischen,
   - Extrudieren und
   - Sphäronisieren,
(iii) Trocknung im Wirbelschichttrockner,
   - Befüllung und
   - Trocknung.

Die einzelnen Schritte sind ausführlich in der folgenden Literatur beschrieben: H. SUCKER, P. FUCHS und P. SPEISER: Pharmazeutische Technologie, Georg Thieme Verlag, Stuttgart, New York, 2. Auflage, 1991.
Zu beachten ist, daß die Pelletierung im Schritt (ii) diskontinuierlich erfolgt, das heißt, die wirkstoffhaltige Pulvermischung mit 0,1% Iloprost wird zyklusweise in circa 5 kg Portionen prozessiert. Die feuchten Rohpellet-Zyklen werden im Wirbelschichttrockner zunächst nacheinander gesammelt. Nachdem der letzte Zyklus im Trockner eintrifft, beginnt die abschließende Trocknung.

Bei dem zuvor genannten Herstellungsverfahren trat neben dem reinen Iloprost bisweilen auch die Verunreinigung Methano - diol - keton auf. Dieses entspricht der Nomenklatur mit dem Namen (1S,2R,3R,5R)-3-Hydroxy-2-[(E)-(3S,4RS)-3-hydroxy-4-methyl-1-octen-6-inyl-bicyclo[3.3.0]octan-7-on. Es handelt sich dabei um ein Zersetzungsprodukt, das durch Oxidation entsteht. Verschiedene Parameter waren im Falle eines Auftretens für die Menge des Zersetzungsproduktes von Bedeutung. Im befeuchteten Zustand war die Oxidation von dem Wassergehalt und von der Inkubationszeit abhängig. Es wurde gesehen, daß die Temperatur eine Rolle spielen kann. Auch war offensichtlich, daß der Wirkstoff in der Mischung mit Avicel und Lactose, nicht jedoch der Wirkstoff alleine anfällig war. Obwohl gleiche Parameter eingehalten wurden, ergaben sich unterschiedliche Mengen an Zersetzungsprodukt, welches bisweilen unterhalb der Nachweisgrenze von < 0,1 % blieb. So waren bisweilen Werte zwischen 0,6% bis 4% üblich. Dadurch wären die Erfordernisse der Reinheit nicht immer erfüllt.

### Aufgabe und Lösung

Es stellt sich somit die Aufgabe, einen Oxidationsschutz bei der Herstellung, insbesondere bei der Verarbeitung von Prostan - Derivaten anzubieten, wobei die Oxidationsprodukte vermieden oder in ihrer Konzentration deutlich reduziert werden.

Die Aufgabe wird gelöst durch Verwendung von einem Aktivkohlefilter als Oxidationsinhibitor für die Umgebungsluft bei bei der Verarbeitung von Prostan-Derivaten, wobei der Oxidationsinhibitor Ozon aus der Pozeßluft vor Kontakt mit den Prostan-Derivaten entfernt.

### Oxidationsinhibitoren

Ein Oxidationsinhibitor kann
eine Inertgasatmosphäre,
ein geschlossener Kreislauf aus synthetischer Luft,
ein Metallkatalysator,
eine Bestrahlungsvorrichtung mit ultraviolettem Licht,
eine Heizvorrichtung mit Temperaturen von mindestens 250 °C und eine Abkühlvorrichtung der Prozeßluft, oder
ein Aktivkohlefilter sein, durch den die Prozeßluft strömt.
Auch Kombinationen aus Oxidationsinhibitoren sind möglich.
Die Oxidationsinhibitoren werden wie folgt charakterisiert:
Eine Inertgasatmosphäre kann aus einer Stickstoffatmosphäre oder auch aus einem Edelgas bestehen.
Synthetische Luft wird als Kreislauf geführt, wobei das bei der Trocknung anfallende Wasser entfernt werden muß. Die Zusammensetzung der Prozeßluft kann dabei im wesentlichen der atmosphärischen Luft mit Ausnahme von Ozon entsprechen.
Als Metallkatalysatoren bieten sich Platin, Kupfer oder Magnesiumoxid an.
Die Bestrahlung mit ultraviolettem Licht erfolgt bevorzugt bei 254 nm.
Die Prozeßluft wird in der Heizvorrichtung bevorzugt auf 300 °C erwärmt. Dabei muß diese Luft anschließend wieder in der Abkühlvorrichtung abgekühlt werden, Beide Prozesse werden bevorzugt in einem Wärmeaustauscher vorgenommen.

### Vorteile

Die unterschiedlichen Mengen an Zersetzungsprodukt bei sonst gleichen Parametern wurden zufällig als eine Abhängigkeit von dem Ozongehalt in der Außenluft festgestellt. Dabei ist anzumerken, daß eine Überwachung der Ozonkonzentration in dem pharmazeutischen Bereich unüblich ist. Derartige Überwachungen sind noch nicht beschrieben worden. Erschwerend kommt hinzu, daß die Zersetzungsprodukte nur dann auftreten, wenn ein Schwellenwert von etwa 20 µg Ozon / m³ Umluft bei der Herstellung überschritten wird. Hierdurch war kein klarer Zusammenhang zwischen der Ozonkonzentration und der Außenluft zu sehen, so daß eine technische Lösung, die befriedigend die Zersetzungsprodukte unterdrücken kann, nicht in Aussicht gestellt werden konnte.

### Zustand der Prostan-Derivate

Bevorzugt ist die Verwendung eines Aktivkohlefilters als Oxidationsinhibitor, wobei die Prostan-Derivate mit Cyclodextrin vermengt sind.
Es hat sich gezeigt, daß die Oxidation verstärkt dann auftritt, wenn die Prostan-Derivate mit Cyclodextrin vermischt sind. Es ist jedoch bekannt, daß gerade die Komplexe aus Prostan-Derivaten und Cyclodextrin besonders stabil gegenüber einer Oxidation sind. Hier wird somit ein sonst übliches Prinzip durchbrochen.

Bevorzugt ist die Verwendung eines Aktivkohlefilters als Oxidationsinhibitor, wobei die Prostan-Derivate sich in einer feuchten Phase befinden.
Die Anwesenheit von Wasser führt zu einer erhöhten Oxidation. Gerade in diesem Falle läuft die Oxidation heftiger ab. Dadurch erschwert sich jedoch die Handhabung des gesamten Prozesses. Die Feuchte in den Prostan-Derivaten und bevorzugt in den Komplexen aus Prostan-Derivaten und Cyclodextrin muß entfernt werden. Hierfür sind Kühlfallen oder andere Vorrichtung notwendig, um den Wasserdampf aus der Prozeßluft zu entfernen.

Bevorzugt ist ein Aktivkohlefilter mit den folgenden Eigenschaften: (i) geringe Kontaktzeiten, (ii) möglichst geringen Druckabfall durch das Filter, (iii) Aktivkohle hat hydrophobe Eigenschaften, und (iv) die Filter besitzen hohe Standzeiten.

### Prostan - Derivate

### Bevorzugt sind Prostan - Derivaten der allgemeinen Formel I oder Formel II

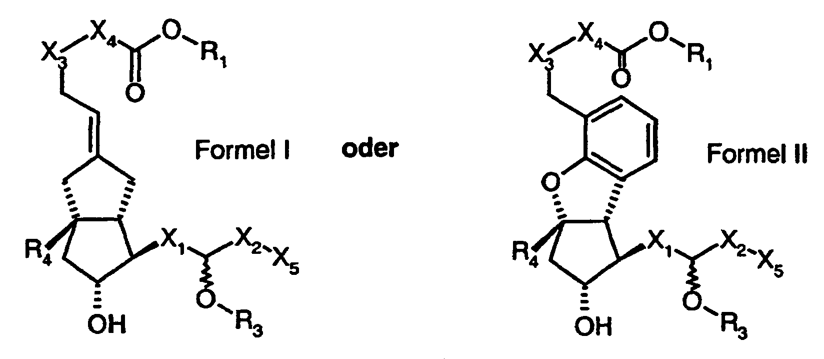

worin
- X₁: ein -CH₂CH₂- ; trans -CH=CH- oder -C ≡ C- ist,
- X₂: eine geradkettige oder verzweigte gesättigte Alkylengruppe mit 1 bis 6 Kohlenstoff - Atomen ist,
- X₃: ein -O- oder -CH₂- ist,
- X₄: ein -CH₂- oder -[CH₂]₃- ist,
- X₅: ein -H oder -C ≡C- R₂ ist;
- R₁: ein Wasserstoff-Atom, eine Alkylgruppe mit 1 bis 6 Kohlenstoffatomen, eine Cycloalkylgruppe mit 5 oder 6 Kohlenstoff-Atomen oder Phenylgruppe ist,
- R₂: eine gerad- oder verzweigtkettige, gesättigte oder ungesättigte Alkylgruppe mit 1 bis 6 Kohlenstoff-Atomen ist,
- R₃: ein Wasserstoff-Atom, ein Acylrest mit 1 bis 4 Kohlenstoff-Atomen oder ein Benzoylrest ist und
- R₄: ein -H oder -CH₃ ist;
wobei die -O- R₃-Gruppe α- oder β- ständig ist,
und deren Salze mit physiologisch verträglichen Basen, falls R₁ die Bedeutung eines Wasserstoff-Atoms hat.

X₂ steht für geradkettige oder verzweigte gesättigte Alkylengruppen mit 1 bis 6 Kohlenstoff-Atomen, so beispielsweise Methylen, Ethylen, Propylen, Isopropyten, wobei die Methylgruppe mit dem ersten oder zweiten Kohlenstoff-Atom des Ethylen, gerechnet von der Gruppe A, verbunden ist; Butylen, Methylpropylen, Ethylethylen, Dimethylethylen, wobei die Methyl- oder Ethylgruppen beliebig mit der Alkytenkette verbunden ist; Pentyl, Methylbutylen, Dimethylpropylen, Ethylpropylen, Methylethylethylen, wobei die Methyl- oder Ethylgruppen beliebig mit der Alkylenkette verbunden ist; Hexylen, Methylpentylen, Dimethylbutylen, Methylethylpropylen, wobei die Methyl- oder Ethylgruppe beliebig mit der Alkylenkette verbunden ist.

Die Alkylgruppen R₁ umfaßt gerade oder verzweigte Alkylgruppen mit 1 bis 6 Kohlenstoff-Atomen, wie beispielsweise Methyl, Ethyl, Propyl, Butyl, Isobutyl, tert.-Butyl, Pentyl, Neopentyl, Hexyl.
Die Cycloalkylgruppe R₁ kann im Ring 5 oder 6 Kohlenstoff-Atome enthalten.

Die Alkylgruppe R₂ kann aus gerad- oder verzweigtkettigen, gesättigten oder ungesättigten Alkylresten mit 1 bis 6 Kohlenstoff-Atomen bestehen, vorzugsweise sind die Alkylreste gesättigt. Beispielsweise genannt seien Methyl-, Ethyl-, Propyl-, Butyl-, Isobutyl-, tert.-Butyl-, Pentyl-, Hexyl-, Butenyl-, Isobutenyl-, Propenyl-, Pentenyl- oder Hexenyl-Gruppen.

Die Acylgruppe R₃ kann aus einer gerad- oder verzweigtkettigen Acylgruppe mit 1 bis 4 Kohlenstoff-Atomen bestehen, wie beispielsweise Acetyl, Propionyl, Butyryl oder Isobutyryl.

Zur Salzbildung mit den freien Säuren sind anorganische und organische Basen geeignet, wie sie dem Fachmann zur Bildung physiologisch verträglicher Salze bekannt sind. Beispielsweise seien genannt: Alkalihydroxide, wie Natrium- und Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin u.s.w. Die β - Cyclodextrinclathrat - Bildung erfolgt entsprechend EP 0 259 468.
Bevorzugt ist das Prostan-Derivat 5-(*E*)-(1*S*,5*S*,6*R*,7*R*)-7-hydroxy-6-[(*E*)-(3*S*,4R*S*)-3-hydroxy-4-methyl-1-octan-6-inyl]bicyclo[3.3.0]octan-3-yliden-pentansäure. Am meisten bevorzugt ist die zuvor genannte Verbindung als β-Cyclodextrin-Einschlußverbindung.

Bevorzugt ist die Verwendung bis zu einer Ozonkonzentration von 20 µg/m³ in der Umluft bei der Herstellung. Nicht die Außenluft ist allein entscheidend. Während des Ansaugens der Luft aus der Außenluft wird Ozon abgebaut, was allein durch Kontakt mit festen Gegenständen erfolgt.

### Vorteile des Aktivkohlefilters

Aktivkohle als Oxidationsschutz zu wählen, war nicht selbstverständlich und naheliegend, wie die ansonsten nicht so erfolgreichen Versuche, den Ozongehalt zu reduzieren, zeigten.

Als erste Lösung wurde eine Stickstoffatmosphäre vorgeschlagen, die aber aus technischen Gründen noch Restmengen an Sauerstoff, das heißt auch anteilsmäßig Ozon, enthält. Die Lösung war nicht befriedigend. Weitere Beseitigung an Sauerstoff wäre in dieser Situation sehr kostenintensiv gewesen. Darüber hinaus kann die Stickstoffatmosphäre nur sinnvoll in einem Umluftbetrieb verwirklicht werden. Dabei besteht wegen der Kreislaufführung der Luft eine erhöhte Gefahr der Kreuz - Kontamination.

Als zweite Lösung wurde vorgeschlagen, die Anlage allein mit synthetischer Luft in einem Kreislauf zu fahren. Die Lösung wäre in diesem Fall ebenfalls sehr kostenintensiv. Wie zuvor schon dargestellt besteht auch in diesem Fall die Gefahr, daß eine Kreuz - Kontamination auftritt.

Als dritte Lösung wurden Metallkatalysatoren aus Platin, Kupfer oder Manganoxid eingesetzt. Hierzu haben die Fachleute und Experten geraten. Die Ozonreduktion war jedoch nicht befriedigend. Auch in diesem Fall hätte man zu einer kostenaufwendigen Lösung greifen müssen. Darüber hinaus ist der Einsatz der angebotenen Metallkatalysatoren mit einem hohen Druckverlust verbunden. Dieser wirkt sich durch die Verschiebung des Druckpotentials in der Anlage negativ auf die Befüllung des Trockners aus.

Als vierte Lösung wurde eine Bestrahlung mit ultraviolettem Licht vorgeschlagen. Hierbei besteht die Gefahr, daß weitere Sauerstoff-Radikale entstehen, die anschließend ebenfalls entfernt werden müssen. Auch diese Lösung entpuppte sich als nicht kostengünstig.

In einer fünfte Lösung wurde Luft auf 300 °C erhitzt. Dabei wird Ozon thermisch zersetzt. Ungünstig ist, daß die Luft wieder abgekühlt werden muß, um eine Temperatur von etwa 40 °C und weniger zu haben. Nachteilig ist, daß in der Lüftungszentrale nicht der erforderliche Platzbedarf für die Aggregate vorhanden ist.

Die Lösung mit Aktivkohlefilter, war nicht naheliegend, da die Fachleute davon abgeraten haben, Aktivkohle einzusetzen, weil der Druckabfall durch den Aktivkohlefilter als zu hoch eingeschätzt wurde. Dabei ist wichtig, darauf hinzuweisen, daß durch den Wirbelschichttrockner, die Luft gesaugt, nicht gedrückt wird. Der Ventilator oder die Turbine wird als Verunreinigungsquelle dadurch ausgeschlossen. Außerdem neigen diese Filter zur Abrasion von Kohlepartikeln, die in das Produktbett gelangen können. Weiterhin muß über längere Standzeiten durch die eintretende feuchte Außenluft mit mikrobieller Kontamination gerechnet werden.
Dennoch stellt sich diese Lösung als die am besten funktionierende Lösung dar.

### Verfahren zur Herstellung der Prostan - Derivate

Die Erfindung umfaßt weiterhin ein Verfahren zur Herstellung, insbesondere Verarbeitung von Medikamenten, die Prostan-Derivate enthalten, unter erfindungsgemäßen Verwendung von einem Aktivkohlefilter als Oxidationsinhibitor, die folgenden Schritte umfassend:
(i) Mischen von dem Wirkstoff Prostan-Derivat-Cyclodextrin und den Hilfsstoffen Lactose und Avicel,
(ii) Pelletierung mit Wasser, bevorzugt mit 15 bis 25% Wasser, mehr bevorzugt mit 20 - 21 % Wasser,
   - Schnellmischen,
   - Extrudieren und
   - Sphäronisieren,
(iii) Trocknung im Wirbelschichttrockner,
   - Befüllung und
   - Trocknung.

Bevorzugt ist ein Verfahren zur Herstellung von Medikamenten, die Prostan-Derivate enthalten, unter erfindungsgemäßer Verwendung von einem Aktivkohlefilter als Oxidationsinhibitor, die folgenden Schritte umfassend:
(i) Mischen von dem Wirkstoff Iloprost-β-Cyclodextrin, der die systematische Bezeichnung 5-(*E*)-(1*S*,5*S*,6*R*,7*R*)-7-hydroxy-6-[(*E*)-(3*S*,4R*S*)-3-hydroxy-4-methy)-1-octen-6-inyl]bicyclo[3.3.0]octan-3-ylidenpentansäure-β-Cyclodextrin-Einschlußverbindung trägt, und den Hilfsstoffen Lactose und Avicel,
(ii) Pelletierung mit Wasser, bevorzugt mit 15 bis 25% Wasser, mehr bevorzugt mit 20 - 21 % Wasser,
   - Schnellmischen,
   - Extrudieren und
   - Sphäronisieren,
(iii) Trocknung im Wirbelschichttrockner,
   - Befüllung und
   - Trocknung.

### Beispiel

Die Überlegenheit der Erfindung wird durch Beispiele belegt. Dabei werden Versuchsansätze mit und ohne Aktivkohlefilter ausgeführt. Um die Wirksamkeit der technischen Lösung zu testen, wird artifiziell eine Prozeßluft mit einem Ozongehalt von 350 ± 25 µg/m³ hergestellt, die zum einen direkt und zum anderen nach der Passage durch einen Aktivkohlefilter zu dem Wirkstoff gelangt. Dabei wird eine Luftmenge von 0,8 m³/sec durch das Filter befördert.
Wird kein Filter eingesetzt, weist die Prozeßluft im Kontaktbereich mit dem Wirkstoff einen Gehalt von 350 ± 25 µg Ozon / m³ Luft auf. Es entstehen dabei 5% Zersetzungsprodukte (w/w).
Wird ein Aktivkohlefilter mit den folgenden Eigenschaften verwendet, wird der Ozongehalt auf 8 ± 0,5 µg Ozon / m³ Luft im Kontaktbereich mit dem Wirkstoff reduziert. Eigenschaften des Aktivkohlefilters: (i) geringe Kontaktzeiten, (ii) möglichst geringer Druckabfall durch das Filter, (iii) Aktivkohle hat hydrophobe Eigenschaften, und (iv) die Filter besitzen hohe Standzeiten.
Das Filter ist ein Standard - Aktivkohlefilter, verpreßt aus zylindrischen Formlingen mit einem Durchmesser von etwa 3 mm, die als Filterpatronen eingesetzt werden. Dadurch entstehen 0,1 % und weniger Zersetzungsprodukte (w/w). Ein solcher Betrag an Zersetzungsprodukten ist pharmakologisch unbedenklich.
Zur Zeit wird nach diesem Verfahren der Wirkstoff 5-(*E*)-(1*S*,5*S*,6*R*,7*R*)-7-hydroxy-6-[(*E*)-(3*S*,4R*S*)-3-hydroxy-4-methyl-1-octen-6-inyl]bicyclo[3.3.0]octan-3-ylidenpentansäure als β-Cyclodextrin-Einschlußverbindung hergestellt

## Patentansprüche

1. Verwendung von einem Aktivkohlefilter als Oxidationsinhibitor für die Umgebungsluft bei der Verarbeitung von Prostan-Derivaten, wobei der Aktivkohlefilter, durch den die Umgebungsluft strömt, Ozon aus der Prozeßluft vor Kontakt mit den Prostan-Derivaten entfernt.

2. Verwendung von einem Aktivkohlefilter gemäß Anspruch 1, wobei die Prostan-Derivate mit Cyclodextrin vermengt sind.

3. Verwendung von einem Aktivkohlefilter gemäß Anspruch 1, wobei sich die Prostan-Derivate in einer feuchten Phase befinden.

4. Verwendung von einem Aktivkohlefilter nach Anspruch 1, wobei ein Aktivkohlefilter mit den folgenden Eigenschaften eingesetzt wird: (i) geringe Kontaktzeiten, (ii) möglichst geringer Druckabfall durch das Filter, (iii) Aktivkohle hat hydrophobe Eigenschaften, und (iv) die Filter besitzen hohe Standzeiten.

5. Verwendung von einem Aktivkohlefilter nach Anspruch 1, wobei das Prostan-Derivat 5-(*E*)-(1*S*,5*S*,6*R*,7*R*)-7-hydroxy-6-[(*E*)-(3*S*,4R*S*)-3-hydroxy-4-methyl-1-octen-6-inyl]bicyclo[3.3.0]octan-3-yliden-pentansäure ist.

6. Verwendung von einem Aktivkohlefilter gemäß Anspruch 5, wobei das Prostan-Derivat als β-Cyclodextrin-Einschlußverbindung vorliegt.

7. Verfahren zur Herstellung von Medikamenten, die Prostan-Derivate enthalten, unter Verwendung von einem Aktivkohlefilter als Oxidationsinhibitor nach einem der vorherigen Ansprüche, die folgenden Schritte umfassend:
(i) Mischen von dem Wirkstoff Prostan-Derivat-Cyclodextrin-Einschlußverbindung und den Hilfsstoffen Lactose und Avicel,
(ii) Pelletierung mit Wasser,
- Schnellmischen,
- Extrudieren und
- Sphäronisieren,
(iii) Trocknung im Wirbelschichttrockner,
- Befüllung und
- Trocknung.

8. Verfahren zur Herstellung von Medikamenten gemäß Anspruch 7, **dadurch gekennzeichnet, dass** der Wirkstoff in Schritt (i) Iloprost-β-Cyclodextrin-Einschlußverbindung ist,
wobei die systematische Bezeichnung von Iloprost 5-(*E*)-(1*S*,5*S*,6*R*,7*R*)-7-hydroxy-6-[(*E*)-(3*S*,4R*S*)-3-hydroxy-4-methyl-1-octen-6-inyl]bicyclo[3.3.0]octan-3-ylidenpentansäure ist.

## Claims

1. Use of an activated-carbon filter as an oxidation inhibitor for ambient air in the processing of prostane derivatives, wherein the activated-carbon filter through which the ambient air follows removes ozone from the process air before contact with the prostane derivatives.

2. Use of an activated-carbon filter as per Claim 1, wherein the prostane derivatives are mixed with cyclodextrin.

3. Use of an activated-carbon filter as per Claim 1, wherein the prostane derivatives are in a moist phase.

4. Use of an activated-carbon filter according to Claim 1, wherein the activated-carbon filter used has the following properties: (i) short contact times, (ii) very small pressure drop through the filter, (iii) activated carbon has hydrophobic properties, and (iv) the filters have long service lives.

5. Use of an activated-carbon filter according to Claim 1, wherein the prostane derivative is 5-(E)-(1S,5S,6R,7R)-7-hydroxy-6-[(E)-(3S,4RS)-3-hydroxy-4-methyl-1-octen-6-ynyl]bicycle[3.3.0]octan-3-ylidenepentanoic acid.

6. Use of an activated-carbon filter as per Claim 5, wherein the prostane derivative is present as a β-cyclodextrin inclusion compound.

7. Process for manufacturing medicaments which contain prostane derivatives by using an activated-carbon filter as an oxidation inhibitor according to any one of the preceding claims, which comprises the following steps:
(i) mixing of the active ingredient prostane derivative-cyclodextrin inclusion compound and the adjuvants lactose and Avicel,
(ii) pelletizing with water,
- high-speed mixing,
- extruding and
- spheronizing,
(iii) drying in a fluidized-bed dryer,
- filling and
- drying.

8. Process for manufacturing medicaments as per Claim 7, **characterized in that** the active ingredient in step (i) is iloprost-β-cyclodextrin inclusion compound, the systematic name of ilosprost being 5-(E)-(1S,5S,6R,7R)-7-hydroxy-6-[(E)-(3S,4RS)-3-hydroxy-4-methyl-1-octen-6-ynyl]bicycle[3.3.0]octan-3-ylidenepentanoic acid.

## Revendications

1. Utilisation d'un filtre à charbon actif en tant qu'agent inhibiteur de l'oxydation pour l'air environnant lors de la mise en oeuvre de dérivés du prostane, le filtre à charbon actif, à travers lequel l'air environnant s'écoule, enlevant l'ozone hors de l'air de processus avant le contact avec les dérivés du prostane.

2. Utilisation d'un filtre à charbon actif selon la revendication 1, les dérivés du prostane étant mélangés à de la cyclodextrine.

3. Utilisation d'un filtre à charbon actif selon la revendication 1, les dérivés du prostane se trouvant dans une phase humide.

4. Utilisation d'un filtre à charbon actif selon la revendication 1, un filtre à charbon actif ayant les propriétés suivantes étant utilisé : (i) des temps de contact faibles, (ii) une chute de pression la plus faible possible à travers le filtre, (iii) le charbon actif a des propriétés hydrophobes et (iv) les filtres possèdent des durées de vie élevées.

5. Utilisation d'un filtre à charbon actif selon la revendication 1, le dérivé du prostane étant l'acide 5-(E)-(1S,5S,6R,7R)-7-hydroxy-6-[(E)-(3S,4RS)-3-hydroxy-4-méthyl-1-octén-6-inyl]bicyclo[3.3.0]octan-3-ylidène-pentanoïque.

6. Utilisation d'un filtre à charbon actif selon la revendication 5, le dérivé du prostane étant présent en tant que composé d'inclusion de la β-cyclodextrine.

7. Procédé en vue de la préparation de médicaments, qui contiennent des dérivés du prostane, avec utilisation d'un filtre à charbon actif en tant qu'agent inhibiteur de l'oxydation selon l'une quelconque des revendications précédentes, qui comprend les étapes suivantes :
(i) le mélange de l'agent actif du composé d'inclusion dérivé du prostane - cyclodextrine et des adjuvants lactose et avicel,
(ii) le bouletage à l'aide d'eau,
- le mélange rapide,
- l'extrusion et
- la mise sous forme de sphères,
(iii) le séchage dans le sécheur à lit fluidisé,
- le remplissage et
- le séchage.

8. Procédé de fabrication de médicaments selon la revendication 7, **caractérisé en ce que** l'agent actif dans l'étape (i) est le composé d'inclusion iloprost-β-cyclodextrine, la désignation systématique d'iloprost étant l'acide 5-(E)-(1S,5S,6R,7R)-7-hydroxy-6-[(E)-(3S,4RS)-3-hydroxy-4-méthyl-1-octén-6-inyl]bicyclo[3.3.0]octan-3-ylidène-pentanoïque.
